# FASCICULE DE BREVET EUROPEEN

(11) **EP 1 453 796 B1**
(45) Date de publication et mention de la délivrance du brevet: **03.03.2010**
(21) Numéro de dépôt: 02799764.2
(22) Date de dépôt: 02.12.2002
(51) Int. Cl.: C07C 311/08, C07D 319/18, A61K 31/18, A61P 1/04, A61P 3/04, A61P 3/10, A61P 15/06, A61P 25/24, A61P 27/06

(54) **PROPANOLAMINOMETHYLTETRALINES, LEUR PREPARATION ET COMPOSITION PHARMACEUTIQUES EN CONTENANT**
PROPANOLAMINOMETHYLTETRALINE, IHRE HERSTELLUNG UND PHARMAZEUTISCHE ZUSAMMENSTELLUNGEN
PROPANOLAMINOMETHYLTETRALINES, THEIR PREPARATION AND PHARMACEUTICAL COMPOSITION COMPRISING SAME

(30) Priorité: 05.12.2001 FR 0115684
(43) Date de publication de la demande: 08.09.2004
(73) Titulaire: Sanofi-Aventis, 75013 Paris (FR)
(72) Inventeur: BARZAGHI, Laura, 20052 Monza (IT); CECCHI, Roberto, I-26900 Lodi (IT); VIVIANI, Nunzia, I-22063 Cantu' (Como) (IT)
(74) Mandataire: Varady, Peter
(86) Numéro de dépôt international: PCT/FR2002/004129
(87) Numéro de publication internationale: WO 2003/048117

(56) Documents cités:
- EP-A- 0 375 560
- US-A- 4 004 028

## Description

La présente invention concerne de nouvelles phénoxypropanolamines, les compositions pharmaceutiques les contenant, un procédé pour leur préparation et des intermédiaires dans ce procédé.

WO99/51564 décrit des dérivés de propanolamine ayant une activité agoniste vis-à-vis des récepteurs bêta-3 adrénergiques et étant susceptibles d'être utilisés dans le traitement de nombreuses maladies telles que l'ulcère, la pancréatite, l'obésité, l'incontinence urinaire et la pollakiurie.

EP 0 375 560 décrit des aryloxypropanolaminotétralines ayant également une activité vis-à-vis des récepteurs bêta-3 adrénergiques mais exerçant un effet antagoniste sur ces récepteurs.

Il a été maintenant trouvé que certaines phénoxypropanolaminotétralines ayant une structure proche des composés décrits dans EP 0 375 560 possèdent une activité agoniste vis-à-vis des récepteurs bêta-3 adrénergiques.

Ainsi, la présente invention concerne, selon un de ses aspects, des phénoxypropanolamines de formule générale (I) 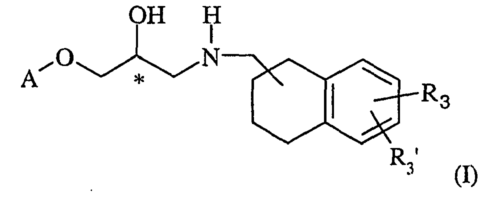 dans laquelle
- A: est un groupe de formule (a) ou (b) 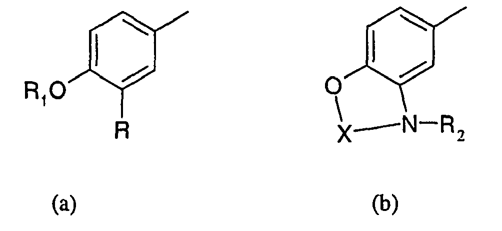
où
- R: représente un atome d'hydrogène ou d'halogène, un groupe -S(O)₂(C₁-C₄)Alk où z est 0, 1, ou 2, un groupe -NHSO₂(C₁-C₄)Alk, -SO₂NH(C₁-C₄)Alk, -NHSO₂-(C₁-C₄)Alk-phényle ou -NHSO₂-phényle, ledit phényle pouvant porter un atome d'halogène, un groupe (C₁-C₄)Alk ou un groupe (C₁-C₆)alcoxy ;
- R₁: représente un atome d'hydrogène ou un groupe -(C₁-C₄)Alk, -CO(C₁-C₄)Alk, -(C₁-C₄)Alk-phényle ou -CO-phényle, ledit phényle pouvant porter un atome d'halogène ou un groupe -(C₁-C₄)Alk ou (C₁-C₆)alcoxy;
- R₂: est un atome d'hydrogène, un groupe -SO₂(C₁-C₄)Alk, -SO₂-(C₁-C₄)Alk-phényle, -SO₂-phényle, ou un groupe -(C₁-C₄)Alk;
- X: complète un cycle de 5 à 8 atomes saturé ou insaturé, pouvant porter un ou deux groupes -(C₁-C₄)Alk et/ou un ou deux groupes carbonyles ;
- R₃ et R'₃: représentent chacun indépendamment un atome d'hydrogène ou d'halogène, un groupe -(C₁-C₆)Alk, hydroxy, -CN, -(C₁-C₆)alcoxy, -COR₄ ou -Y-CR₈R₉-COR₄;
- Y: représente O ou CH₂;
- R₄: représente un groupe hydroxy, (C₁-C₆)alcoxy ou -NR₅R₆;
- R₅ et R₆: représentent indépendamment un atome d'hydrogène; un groupe -(C₁-C₄)Alk; aryle ou hétéroaryle éventuellement substitués par un groupe R₇, aralkyle ou hétéroaralkyle éventuellement substitués par un groupe R₇;
ou bien R₅ et R₆, avec l'atome d'azote qui les porte, forment un cycle de 5 à 7 atomes, saturé ou insaturé, éventuellement substitué par un groupe R₇; et
- R₇: représente un atome d'hydrogène ou d'halogène, un groupe hydroxy, -(C₁-C₄)Alk, -(C₁-C₆)alcoxy, -NH(C₁-C₄)Alk, -N(C₁-C₄)Alk₂, -COO(C₁-C₄)Alk, aralkyle ou hétéroaralkyle;
- R₈ et R₉: représentent indépendamment un atome d'hydrogène ou un groupe -(C₁-C₄)Alk;
et leurs sels ou solvates.

Dans la présente description les termes -(C₁-C₄)Alk et -(C₁-C₆)Alk désignent des radicaux monovalents d'un hydrocarbure à chaîne saturée droite ou ramifiée.

Dans la présente description le terme -(C₁-C₆)alcoxy désigne un radical monovalent d'un hydrocarbure à chaîne saturée droite, ramifiée ou cyclique, rattaché au reste de la molécule par un atome d'oxygène.

Des groupes aryles ou hétéroaryles préférés comprennent notamment le phényle, le naphtyle et le pyridyle.

Les groupes aralkyles ou hétéroaralkyles désignent respectivement des groupes aryl-alkyle ou hétéroaryl-alkyle, reliés au reste de la molécule par la chaîne alkylique.

Des groupes aralkyles ou hétéroaralkyles préférés comprennent notamment le benzyle, le naphtylméthyle et le pyridylméthyle.

Les cycles de 5 à 7 atomes, saturés ou insaturés, comprennent notamment la pyrrolidine, la pipéridine, la morpholine et la thiomorpholine.

Des composés préférés sont ceux où A est un groupe de formule (a).

D'autres composés préférés sont ceux où R est un groupe -NHSO₂(C₁-C₄)Alk.

D'autres composés préférés sont ceux où R₁ est un atome d'hydrogène.

D'autres composés préférés sont ceux où R₃ et/ou R'₃ sont un atome d'hydrogène, un groupe hydroxy, (C₁-C₆)alcoxy ou -Y-CR₈R₉-COOCH₂-CH₃.

Les sels des composés de formule (I) selon la présente invention comprennent aussi bien les sels d'addition à des acides minéraux ou organiques pharmaceutiquement acceptables tels que le chlorhydrate, le bromhydrate, le sulfate, l'hydrogénosulfate, le dihydrogénophosphate, le citrate, le maléate, le tartrate, le fumarate, le gluconate, le méthanesulfonate, le 2-naphtalènesulfonate, etc., que les sels d'addition qui permettent une séparation ou une cristallisation convenable des composés de formule (I) tels que le picrate, l'oxalate ou les sels d'addition à des acides optiquement actifs, par exemple les acides camphosulfoniques et les acides mandéliques ou mandéliques substitués.

De plus, lorsque les composés de formule (I) possèdent un groupe carboxy libre les sels comprennent aussi les sels de bases minérales, de préférence celles avec des métaux alcalins tels que le sodium ou le potassium, ou de bases organiques. La tétraline peut être rattachée au groupe amino-méthyl par le carbone en position alpha ou bêta, le carbone de rattachement de la tétraline étant un carbone asymétrique.

Les stéréoisomères optiquement purs, ainsi que les mélanges d'isomères des composés de formule (I), dus aux carbones asymétriques, dans une proportion quelconque, sont partie de la présente invention.

Des composés de formule (I) préférés sont les composés où la configuration du carbone de la propanolamine portant le groupe OH est (S).

Les composés de formule (I) peuvent être préparés en traitant un composé de formule (II) : 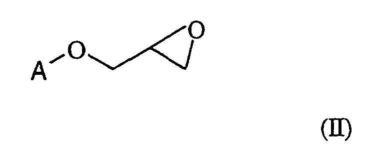 dans laquelle A est tel qu'indiqué ci-dessus, avec une amine de formule (III) 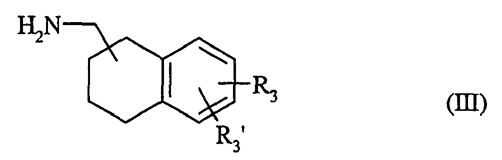 sous forme de base non salifiée, où R₃ et R'₃ sont tels que définis ci-dessus et éventuellement en transformant le composé de formule (I) ainsi obtenu en un de ses sels.

Plus particulièrement, la réaction entre les composés de formule (II) et (III) est réalisée dans un solvant organique, tel qu'un alcool inférieur comme le méthanol, l'éthanol et le propan-2-ol; le diméthylsulfoxyde (DMSO); un éther linéaire ou cyclique; un amide comme le diméthylformamide (DMF) ou le diméthylacétamide; en utilisant préférablement des quantités au moins équimoléculaires des réactifs.

La température de la réaction est comprise entre la température ambiante et la température de reflux du solvant choisi.

Lorsque R₁ représente l'hydrogène, il est préférable de protéger le groupe fonctionnel par un groupe protecteur afin de faciliter la condensation souhaitée.

Comme groupes protecteurs, on peut utiliser les groupes protecteurs usuels pour le groupe hydroxy tels que par exemple le méthoxyéthoxyméthyle (MEM), le triméthylsilyléthoxyméthyle (SEM) ou le benzyle, selon les techniques bien connues.

De la même façon, les autres groupes sensibles éventuellement présents peuvent être protégés selon les méthodes bien connues.

Les époxydes de formule (II) sont des composés connus en littérature ou bien ils peuvent être préparés par des procédés analogues aux procédés décrits dans la littérature.

Les aminotétralines de formule (III) peuvent être préparées selon des modes opératoires connus en littérature, par exemple comme décrit dans EP-A-436435 ou dans EP-A-683236.

Les composés de formule (III) où R₃ et/ou R'₃ sont un groupe -(CH₂)₂COR₄ (R₄ étant tel que défini dans la formule (I), ainsi que leurs sels, sont nouveaux et constituent un autre objet de la présente invention; ces composés peuvent être préparés selon le mode opératoire décrit dans WO01/94307 (Schéma 1).

L'activité des composés de la présente invention vis-à-vis de l'effet bêta-3 a été mise en évidence à l'aide d'essais in vitro sur le colon humain selon la méthode décrite et dans T. Croci et al, Br. J. Pharmacol., 1997, 122: 139P, par L. Manara et al., Gut, 2000, 47:337-342 et dans EP-A-436435. Plus particulièrement, on a constaté que les composés de formule (I) sont beaucoup plus actifs sur le côlon isolé que sur l'oreillette et sur la trachée.

Ces propriétés surprenantes des composés de formule (I) permettent d'envisager leur utilisation comme médicaments à action bêta-3 agoniste.

De plus, les composés de formule (I) sont peu toxiques; notamment, leur toxicité aiguë est compatible avec leur utilisation comme médicaments pour le traitement de maladies dans lesquelles les composés ayant une affinité pour le récepteur bêta-3, notamment les bêta-3 agonistes, trouvent leur application. De telles maladies sont décrites en littérature. Les composés de formule (I), ainsi que leurs sels pharmaceutiquement acceptables, peuvent donc par exemple être indiqués dans le traitement des maladies gastro-intestinales telles que les maladies inflammatoires de l'intestin comme le syndrome du colon irritable (IBD), comme modulateurs de la motricité intestinale, comme lipolytiques, agents anti-obésité, anti-diabétiques, psychotropes, anti-glaucomateux, cicatrisants, anti-dépresseurs, comme inhibiteur des contractions utérines, comme tocolytiques pour prévenir ou retarder les accouchement précoces, pour le traitement et/ou la prophylaxie de la dysménorrhée. En outre les composés de formule (I) peuvent être utilisés dans le traitement de certaines maladies du système nerveux central, telle que par exemple la dépression, ainsi que de certains troubles du système urinaire tel que l'incontinence urinaire.

L'utilisation des composés de formule (I) ci-dessus, ainsi que celle de leurs sels et solvates pharmaceutiquement acceptables pour la préparation de médicaments ci-dessus, constitue un aspect ultérieur de la présente invention.

Pour une telle utilisation, on administre aux mammifères qui nécessitent un tel traitement une quantité efficace d'un composé de formule (I) ou d'un de ses sels et solvates pharmaceutiquement acceptables.

Les composés de formule (I) ci-dessus et leurs sels et solvates pharmaceutiquement acceptables peuvent être utilisés à des doses journalières de 0,01 à 20 mg par kg de poids corporel du mammifère à traiter, de préférence à des doses journalières de 0,1 à 10 mg/kg. Chez l'être humain, la dose peut varier de préférence de 0,5 mg à 1500 mg par jour, notamment de 2,5 à 500 mg selon l'âge du sujet à traiter, le type de traitement, prophylactique ou curatif, et la gravité de l'affection. Les composés de formule (I) sont généralement administrés en unité de dosage de 0,1 à 500 mg, de préférence de 0,5 à 100 mg de principe actif, une à cinq fois par jour.

Lesdites unités de dosage sont de préférence formulées dans des compositions pharmaceutiques dans lesquelles le principe actif est mélangé avec un excipient pharmaceutique.

Ainsi, selon un autre de ses aspects, la présente invention concerne des compositions pharmaceutiques renfermant, en tant que principe actif, un composé de formule (I) ci-dessus ou un de ses sels et solvates pharmaceutiquement acceptables.

Dans les compositions pharmaceutiques de la présente invention pour l'administration orale, sublinguale, sous-cutanée, intramusculaire, intraveineuse, topique, transdermique ou rectale, les ingrédients actifs de formule (I) ci-dessus, leurs sels et solvates pharmaceutiquement acceptables peuvent être administrés sous formes unitaires d'administration, en mélange avec des supports pharmaceutiques classiques, aux animaux et aux êtres humains, pour le traitement des affections susdites. Les formes unitaires d'administration appropriées comprennent les formes par voie orale telles que les comprimés, les gélules, les poudres, les granulés et les solutions ou suspensions orales, les formes d'administration sublinguale et buccale, les formes d'administration sous-cutanée, intramusculaire ou intraveineuse, les formes d'administration locale et les formes d'administration rectale.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, acalorique de préférence, du méthylparaben et du propylparaben comme antiseptiques, ainsi qu'un agent donnant du goût et un colorant approprié.

Les poudres ou les granulés dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration locale, on mélange le principe actif dans un excipient pour la préparation de crèmes ou onguents ou on le dissout dans un véhicule pour l'administration intraoculaire, par exemple sous forme de collyre.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Selon un autre de ses aspects, la présente invention concerne l'utilisation d'un composé de formule (I) ou l'un de ses sels ou solvates pharmaceutiquement acceptables pour la préparation d'un médicament destiné au traitement des pathologies qui sont améliorées par une action bêta-3-agoniste.

Les composés de formule (I) notamment les composés (I) marquées par un isotope, peuvent aussi être utilisés comme outils de laboratoire dans des essais biochimiques.

Les composés de formule (I) se lient au récepteur bêta-3-adrénergique. On peut donc utiliser ces composés dans un essai ordinaire de liaison ("binding"), dans lequel on emploie un tissu organique où ce récepteur est particulièrement abondant, et on mesure la quantité de composé (I) déplacé par un composé test, pour évaluer l'affinité dudit composé vis-à-vis des sites de liaison de ce récepteur particulier.

Un autre objet spécifique de la présente invention est donc un réactif utilisable dans les essais biochimiques, qui comprend au moins un composé de formule (I) convenablement marqué.

Les exemples qui suivent illustrent mieux l'invention.

Tous les spectres RMN, au moins qu'il ne soit autrement indiqué, ont été enregistré à 400 MHz.

Dans la description des HPLC: PS = Phase stationnaire; PM = Phase Mobile; t. = tampon; d = débit ; TR = temps de rétention.

### EXEMPLE 1

### N-[2-hydroxy-5-({(2S)-2-hydroxy-3-[(1,2,3,4-tétra-hydro-2-naphtalènylméthyl)-amino]-propyl}-oxy)-phényl]-méthanesulfonamide

### (a). N-[2-benzyloxy-5-({(2S)-2-hydroxy-3-[(1,2,3,4-tétra-hydro-2-naphtalènylméthyl)-amino]-propyl}-oxy)-phényl]-méthanesulfonamide.

On chauffe au reflux un mélange de 770 mg de 4-benzyloxy-3-(N-tert-butoxycarbonyl-N-méthylsulfonyl-amino)-1-((2S)2,3-époxypropoxy)-benzène (1,71 mmole) et 300 mg (1,8 mmole) de 1,2,3,4-tétra-hydro-naphtalèn-2-ylméthylamine, obtenue comme décrit dans EP-A-436435, dans 20 ml d'éthanol absolu pendant 16 heures. On refroidit le mélange, on y ajoute 3 ml d'une solution d'éthanol saturée en acide chlorhydrique et on chauffe à 50 °C pendant 5 heures. On évapore le solvant et on reprend avec un mélange de 50 ml d'une solution saturée en bicarbonate de sodium et 50 ml d'acétate d'éthyle. On lave la phase organique avec une solution aqueuse saturée en NaCl. On sèche la phase organique, on filtre et on évapore le solvant sous pression réduite. On purifie le produit brut par chromatographie sur colonne de gel de silice en éluant avec un mélange chlorure de méthylène/méthanol/ =95/5. On obtient le composé du titre sous forme de solide vitreux.

### (b). N-[2-hydroxy-5-({(2S)-2-hydroxy-3-[(1,2,3,4-tétra-hydro-2-naphtalènylméthyl)-amino]-propyl}-oxy)-phényl]-méthanesulfonamide

On agite une solution de 400 mg du produit de l'exemple (1a) (0,8 mmole) dans 25 ml d'un mélange d'éthanol et de THF, à la température ambiante et sous atmosphère d'hydrogène, pendant 7 heures en présence de 80 mg de Pd/C à 10%. On filtre le catalyseur, on évapore le solvant sous pression réduite et on purifie le produit brut par chromatographie sur colonne de gel de silice en éluant avec un mélange chlorure de méthylène/méthanol =9/1. On obtient le composé du titre.
Masse = MH⁺ 421
RMN (DMSO + D20 313K); 200 MHz
1.27-1.59 (m; 1H); 1.80-2.26 (m; 2H); 2.34-2.57 (m; 1H); 2.67-2.88 (m; 2H); 2.93 (s; 3H); 2.97-3.13 (m; 3H); 3.13-3.31 (m; 1H); 3.83-3.98 (m; 2H); 6.67 (dd; 1H) Ja 8.8Hz, Jb 2.9Hz; 6.83 (d; 1H) J 8.8Hz; 6.83 (d; 1H) J2.9Hz; 7.06 (s; 4H).

### EXEMPLE 2

### N-{2-hydroxy-5-[((2S)-2-hydroxy-3-{[(7-méthoxy-1,2,3,4-tétra-hydro-2-naphtalènyl)-méthyl]-amino}-propyl)-oxy]-phényl}-méthanesulfonamide

### (a). N-{2-benzyloxy-5-[((2S)-2-hydroxy-3-{[(7-méthoxy-1,2,3,4-tétra-hydro-2-naphtalènyl)-méthyl]-amino}-propyl)-oxy]-phényl}-méthanesulfonamide

En opérant comme dans l'exemple (1a), avec la 7-méthoxy-1,2,3,4-tétra-hydro-naphtalèn-2-ylméthylamine obtenue comme décrit dans EP-A-436435, on obtient le composé du titre sous forme d'un solide vitreux.

### (b). N-{2-hydroxy-5-[((2S)-2-hydroxy-3-{[(7-méthoxy-1,2,3,4-tétra-hydro-2-naphtalènyl)-méthyl]-amino}-propyl)-oxy]-phényl}-méthanesulfonamide

En opérant comme décrit dans l'exemple (1b), avec le produit obtenu à l'étape (2a), on obtient le composé du titre.
Masse = MH⁺ 451
RMN (DMSO + TFA 313K); 200 MHz
1.24-1.52 (m; 1H); 1.91-2.29 (m; 2H); 2.41-2.48 (m; 1H); 2.58-3.29 (m; 7H); 2.92 (s; 3H); 3.66 (s; 3H); 3.74-3.98 (m; 2H); 4.07-4.30 (m; 1H); 6.54-6.73 (m; 3H); 6.73-6.89 (m; 2H); 6.89 7.02 (m; 1H).

### EXEMPLE 3

### N-{2-hydroxy-5-[((2S)-2-hydroxy-3-{[(6-méthoxy-1,2,3,4-tétra-hydro-2-naphtalènyl)-méthyl]-amino}-propyl)-oxy]-phényl}-méthanesulfonamide

### (a). N-{2-benzyloxy-5-[((2S)-2-hydroxy-3-{[(6-méthoxy-1,2,3,4-tétra-hydro-2-naphtalènyl)-méthyl]-amino}-propyl)-oxy]-phényl}-méthanesulfonamide

En opérant comme dans l'exemple (1a), avec la 6-méthoxy-1,2,3,4-tétra-hydro-naphtalèn-2-ylméthylamine obtenue comme décrit dans EP-A-436435, on obtient le composé du titre sous forme d'un solide vitreux.

### (b). N-{2-hydroxy-5-[((2S)-2-hydroxy-3-{[(6-méthoxy-1,2,3,4-tétra-hydro-2-naphtalènyl)-méthyl]-amino}-propyl)-oxy]-phényl}-méthanesulfonamide

En opérant comme dans l'exemple (1b), avec le produit obtenu à l'étape (3a), on obtient le composé du titre.
Masse = MH⁺ 451
RMN (DMSO 313K)
1.27-1.43 (m; 1H); 1.85-2.02 (m; 2H); 2.35 (dd; 1H) Ja 16Hz, Jb 10Hz; 2.62-2.84 (m; 6H); 2.88 (dd; 1H) Ja 12Hz, Jb 4Hz; 2.95 (s; 3H); 3.70 (s; 3H); 3.76-3.93 (m; 2H); 3.93-4.07 (m; 1H); 6.57-6.71 (m; 3H); 6.81 (d; 1H) J 9Hz; 6.83 (d; 1H) J 3Hz; 6.96 (d; 1H)J 8Hz.
HPLC = PS: SUPELCOSIL LC-ABZ 15 x 0.46 cm. PM: t. KH₂PO₄ 0.02M pH 3.5/CH3CN/MeOH 80:18:2; d = 1ml/min; λ = 205 nm;TR = 9.05.min
HPLC = PS: CHIRALCEL OD-H 25 × 0.46 cm. PM: hexane/éthanol 75:25; d = 0.8 ml/min; λ = 235 nm;TR₁ = 15.07 min (2S, 2R); TR₂ = 19.68.min (2S, 2S).

### EXEMPLE 4

### N-(2-hydroxy-5-{[(2S)-2-hydroxy-3-({[(2R)-6-méthoxy-1,2,3,4-tétra-hydro-2-naphtalènyl]-méthyl}-amino)-propyl]-oxy}-phényl)-méthanesulfonamide

### (a) N-(2-benzyloxy-5-{[(2S)-2-hydroxy-3-({[(2R)-6-méthoxy-1,2,3,4-tétra-hydro-2-naphtalènyl]-méthyl}-amino)-propyl]-oxy}-phényl)-méthanesulfonamide

En opérant comme dans l'exemple (1a), en utilisant la (2R)-6-méthoxy-1,2,3,4-tétra-hydro-naphtalèn-2-yl-méthylamine obtenue comme décrit dans EP-A-683236, on obtient le composé du titre sous forme d'un solide vitreux.

### (b) N-(2-hydroxy-5-{[(2S)-2-hydroxy-3-({[(2R)-6-méthoxy-1,2,3,4-tétra-hydro-2-naphtalènyl]-méthyl}-amino)-propyl]-oxy}-phényl)-méthanesulfonamide

En opérant comme dans l'exemple (1b), avec le produit obtenu à l'étape (4a), on obtient le composé du titre.
Masse = MH⁺ 451
RMN (DMSO 313K)
1.18-1.38 (m; 1H); 1.70-1.98 (m; 2H); 2.26-2.36 (m; 1H); 2.53-2.58 (m; 2H); 2.60-2.82 (m; 5H); 2.94 (s; 3H); 3.63-3.73 (m; 3H); 3.73-3.82 (m; 1H); 3.82-3.91 (m; 1H); 6.63-6.69 (m; 3H); 6.72-6.84 (m; 2H); 6.94 (d; 1H) J 8Hz.
HPLC = PS: CHIRALCEL OD-H 25 × 0.46 cm. PM: hexane/éthanol 75:25; d = 0.8 ml/min; λ = 235 nm;TR₁ = 14.53 min (ee 84.2%).

### EXEMPLE 5

### N-{2-hydroxy-5-[((2S)-2-hydroxy-3-{[(7-hydroxy-1,2,3,4-tétra-hydro-2-naphtalènyl)-méthyl]-amino}-propyl)-oxy]-phényl}-méthanesulfonamide

### (a). N-{2-benzyloxy-5-[((2S)-hydroxy-3-{[(7-hydroxy-1,2,3,4-tétra-hydro-2-naphtalènyl)-méthyl]-amino}-propyl)-oxy]-phényl}-méthanesulfonamide

En opérant comme dans l'exemple (1a), avec la 7-hydroxy-1,2,3,4-tétra-hydro-naphtalèn-2-ylméthylamine obtenue comme décrit dans EP-A-436435, on obtient le composé du titre sous forme d'un solide vitreux.

### (b). N-{2-hydroxy-5-[((2S)-2-hydroxy-3-{[(7-hydroxy-1,2,3,4-tétra-hydro-2-naphtalènyl)-méthyl]-amino}-propyl)-oxy]-phényl}-méthanesulfonamide

En opérant comme dans l'exemple (1b), avec le produit de l'étape (5a), mais en éluant avec un mélange CH₂Cl₂/CH₃OH/NH₄OH = 9/1/0,1, on obtient le composé du titre.
Masse = MH⁺ 437
RMN (DMSO + D20 313K); 300 MHz
1.18-1.44 (m; 1H); 1.64-2.03 (m; 2H); 2.20-2.41 (m; 1H); 2.54-2.85 (m; 6H); 2.93 (s; 3H); 3.72-4.04 (m; 3H); 5.46-5.90 (m; 1H); 6.23-6.56 (m; 2H); 6.56-6.72 (m; 1H); 6.72-7.01 (m; 3H).
HPLC = PS: SUPELCOSIL LC-ABZ 15 × 0.46 cm. PM: t. K₂HPO₄ 0.02M pH 7.0/MeOH 7:3; d = 1ml/min; λ = 295 nm; TR = 35.2 min.

### EXEMPLES 6-14

En suivant le mode opératoire décrit dans l'exemple 1, mais en utilisant les époxydes et les aminométhyltétralines convenables, on obtient les composés rapportés dans le Tableau suivant:

**TABLEAU**

| | |
|---|---|
| **Ex. 6** | 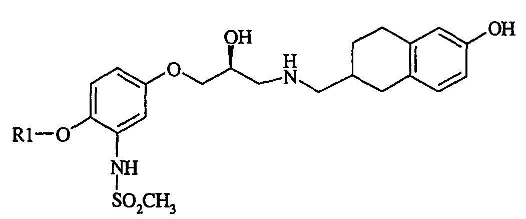 |
| Ex. 6a⁽¹⁾ R₁ = Bn | |
| Ex. 6b | **Masse = MH⁺ 437** |
| R₁ = H | **RMN** (DMSO + D2O 313K) 1.16-1.42 (m; 1H); 1.61-1.98 (m; 2H);2.11-2.34 (m; 1H); 2.41-2.54 (m; 2H); 2.54-2.77 (m; 5H); 2.90 (s; 3H); 3.72-3.80 (m; 1H); 3.80-3.92 (m; 2H); 6.35-6.54 (m; 2H); 6.56 (dd; 1H) Ja 9Hz, Jb 3Hz; 6.76 (d; 1H) J 9Hz; 6.78-6.91 (m; 2H). |
| | **HPLC** = PS: SUPELCOSIL LC-ABZ 15 × 0.46 cm. PM: t. K₂HPO₄ 0.01M pH 7.0/MeOH 65:35; d = 1ml/min; λ = 225 nm; TR = 13.2 min |
| **Ex. 7** | 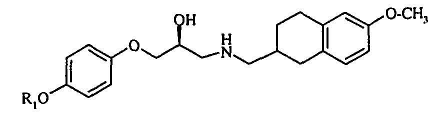 |
| Ex. 7a⁽¹⁾ R₁ = Bn | |
| Ex. 7b | **Masse = MH⁺ 358** |
| R₁ = H | **RMN** (DMSO + D20 313K) 1.17-1.39 (m; 1H); 1.67-1.96 (m; 2H); 2.28 (dd; 1H) Ja 16Hz, Jb 11Hz; 2.52-2.60 (m; 1H); 2.60-2.81 (m; 4H); 3.67 (s; 3H); 3.72-3.91 (m; 3H); 6.55-6.70 (m; 4H); 6.70-6.79 (m; 2H); 6.93 (d; 1H) J 8Hz.. |
| | **HPLC** = PS: SUPELCOSIL LC-ABZ + Plus 15 × 0.46 cm. PM: t. K₂HPO₄ 0.02M pH 7.0/MeOH 1:1; d = 1m1/min; λ = 225 nm; TR = 9.07 min. |
| **Ex. 8** | 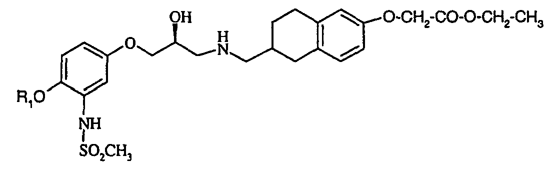 |
| Ex. 8a ⁽¹⁾ R₁ = Bn | |
| Ex. 8b | **Masse = MH⁺ 523** |
| R₁ = H | **RMN** (DMSO 313K) 1.21 (t; 3H) J 7Hz; 1.24-1.38 (m; 1H); 1.73-2.01 (m; 2H); 2.20-2.39 (m; 1H); 2.54-2.83 (m; 7H); 2.94 (s; 3H); 3.73-3.95 (m; 3H); 4.16 (q; 2H) J 7Hz; 4.69 (s; 2H); 6.54-6.69 (m; 3H); 6.79 (d; 1H) J 9Hz; 6.81 (d; 1H) J 3Hz; 6.96 (d; 1H) J 8Hz. |
| | **HPLC** = PS: SUPELCOSIL LC-ABZ + Plus 15 × 0.46 cm. PM: t. K₂HPO₄ 0.02M pH 7.0/MeOH 1:1; d = 1ml/min; λ = 225 nm; TR = 6.3 min. |
| **Ex. 9** | 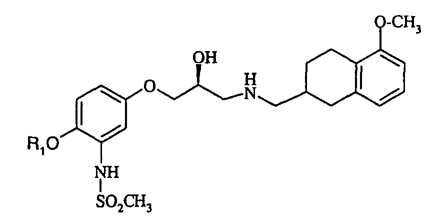 |
| Ex. 9a⁽¹⁾ R₁ = Bn | |
| Ex. 9b | **Masse** = **MH⁺ 451** |
| R₁ = H | **RMN** (DMSO + D2O 313K) 1.17-1.40 (m; 1H); 1.65-1.86 (m; 1H);1.86-2.47 (m; 1H); 2.52-2.85 (m; 6H); 2.92 (s; 3H); 3.73 (s; 3H); 3.734.07 (m; 3H); 6.46-6.88 (m; 5H); 6.88-7.16 (m; 1H). |
| | **HPLC** = PS: SUPELCOSIL LC-ABZ + Plus 15 × 0.46 cm. PM: t. K₂HPO₄ 0.02M pH 7.0/CH₃CN 3:7; d = 1ml/min; λ = 225 nm; TR = 1.8 min. |
| **Ex.10** | 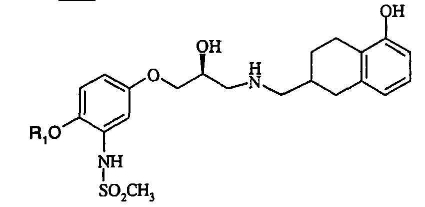 |
| Ex. 10a ⁽¹⁾ R₁ =Bn | |
| Ex. 10b | **Masse = MH⁺ 437** |
| R₁ = H | **RMN** (DMSO + D20 313K) |
| | 1.18-1.47 (m; 1H); 1.65-2.07 (m; 2H); 2.20-2.45 (m; 2H); 2.53-2.85 (m; 6H); 2.90 (s; 3H); 3.74-4.00 (m; 3H); 6.33-6.95 (m; 6H). |
| **Ex. 11** | 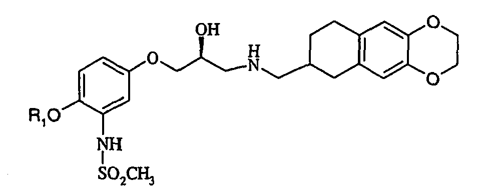 |
| Ex. 11a ⁽²⁾ R₁ = Bn | |
| Ex. 11b | **Masse = MH⁺479** |
| R₁ = H | **RMN** (DMSO 313K) |
| | 1.17-1.34 (m; 1H); 1.68-1.97 (m; 2H); 2.26 (dd; 1H) Ja 16Hz, Jb 10Hz; 2.51-2.78 (m; 7H); 2.94 (s; 3H); 3.70-3.94 (m; 3H); 4.16 (bs; 4H); 6.51 (bs; 2H); 6.62 (dd; 1H) Ja 9Hz, Jb 3Hz; 6.78 (d; 1H) J 8Hz; 6.81 (d; 1H) J 3Hz. **HPLC** = PS: SUPELCOSIL LC-ABZ + Plus 15 × 0.46 cm. PM: t. K₂HPO₄ 0.02M pH 7.0/MeOH 1:1; d = 1m1/min; λ = 225 nm; TR = 5.56 min. |
| **Ex. 12** | 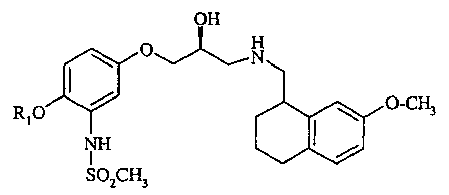 |
| Ex. 12a⁽³⁾ R₁ = Bn | |
| Ex. 12b | **Masse = MH⁺ 451** |
| R₁ = H | **RMN** (DMSO 313K) |
| | 1.52-1.89 (m; 4H); 2.53-2.97 (m; 7H); 2.94 (s; 3H); 3.69 (s; 3H); 3.74-3.82 (m; 1H); 3.82-3.94 (m; 2H); 6.62 (dd; 1H) Ja 9Hz, Jb 3Hz; 6.63 (dd; 1H) Ja 8Hz, Jb 3Hz; 6.73 (m; 2H); 6.81 (dd; 1H) J 3Hz; 6.94 (dd; 1H) J 8Hz. **HPLC** = PS: XTerra^{™} RP18 15 × 0.3 cm. PM: TEA-CH₃COOH pH 10.0/CH3CN 7:3; d = 0.5 ml/min; λ = 225 nm ; TR = 7.14 min. |
| **Ex. 13** | 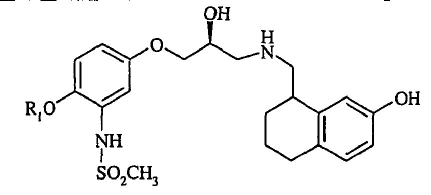 |
| Ex. 13a⁽⁴⁾ R₁ = Bn | |
| Ex. 13b | **Masse = MH⁺ 437** |
| R₁ = H | **RMN** (DMSO 313K) |
| | 1.45-1.89 (m; 4H); 2.53-2.82 (m; 7H); 2.94 (s; 3H); 3.17 (s; 1H); 3.72-3.82 (m; 1H); 3.82-3.93 (m; 2H); 6.49 (dd; 1H) Ja 8Hz, Jb 2Hz; 6.56-6.67 (m; 2H); 6.78 (d; 1H) J 9Hz; 6.80-6.87 (m; 2H). **HPLC** = PS: SUPELCOSIL LC-ABZ + Plus 15 × 0.46 cm. PM: t. K₂HPO₄ 0.02M pH 7.0/CH₃CN 3:7; d = 1ml/min; λ = 225 nm; TR =1.97 min. |
| **Ex. 14** | 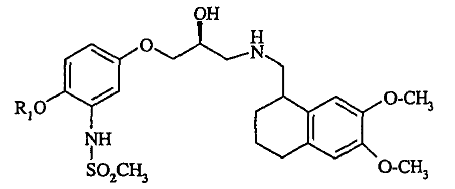 |
| Ex. 14a ⁽⁵⁾ R₁ = Bn | |
| Ex. 14b | **Masse = MH⁺ 481** |
| R₁ = H | **RMN** (DMSO 313K) |
| | 1.50-1.88 (m; 4H); 2.54-2.84 (m; 7H); 2.93 (s; 3H); 3.69 (bs; 6H); 3.74-3.94 (m; 3H); 6.59 (s; 1H); 6.52-6.65 (m; 1H); 6.77 (d; 1H) J 9Hz; 6.79 (s; 1H); 6.81 (d; 1H) J 3Hz. **HPLC** = PS: SUPELCOSIL LC-ABZ + Plus 15 × 0.46 cm. PM: t. K₂HPO₄ 0.02M pH 7.0/CH₃CN 7:3; d = 1ml/min; λ = 225 nm ; TR = 4.82 min. |

| | |
|---|---|
| Notes: Bn = benzyle ⁽¹⁾ = aminométhyltétraline décrite dans EP-A-436435. ⁽²⁾ = aminométhyltétraline préparée selon le mode opératoire décrit dans EP-A-436435 à partir de la 2,3,8,9-tétra-hydro-naphto[2,3-b]-1,4-dioxin-6-one décrite dans Biorg. Med. Chem. Lett. 6 (10):1071, 1966. ⁽³⁾ = aminométhyltétraline décrite dans J. Med. Chem. 18 (12): 1266, 1975. ⁽⁴⁾ = aminométhyltétraline obtenue par déméthylation de l'aminométhyltétraline ⁽³⁾ ci-dessus. ⁽⁵⁾ = aminométhyltétraline décrite dans J. Med. Chem. 26 (6): 813, 1983. | |

## Revendications

1. Composé de formule générale (I) 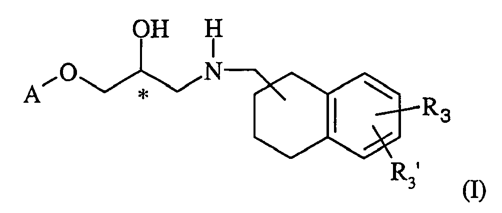 dans laquelle
A est un groupe de formule (a) ou (b) 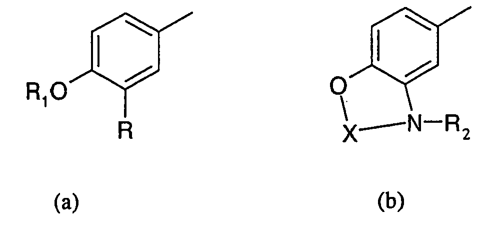
où
R représente un atome d'hydrogène ou d'halogène, un groupe -S(O)_{z}(C₁-C₄)Alk où z est 0, 1, ou 2, un groupe -NHSO₂(C₁-C₄)Alk, -SO₂NH(C₁-C₄)Alk, -NHSO₂-(C₁-C₄)Alk-phényle ou -NHSO₂-phényle, ledit phényle pouvant porter un atome d'halogène, un groupe (C₁-C₄)Alk ou un groupe (C₁-C₆)alcoxy ;
R₁ représente un atome d'hydrogène ou un groupe -(C₁-C₄)Alk, -CO(C₁-C₄)Alk, -(C₁-C₄)Alk-phényle ou -CO-phényle, ledit phényle pouvant porter un atome d'halogène ou un groupe -(C₁-C₄)Alk ou (C₁-C₆)alcoxy;
R₂ est un atome d'hydrogène, un groupe -SO₂(C₁-C₄)Alk, -SO₂-(C₁-C₄)Alk-phényle, -SO₂-phényle, ou un groupe -(C₁-C₄)Alk;
X complète un cycle de 5 à 8 atomes saturé ou insaturé, pouvant porter un ou deux groupes -(C₁-C₄)Alk et/ou un ou deux groupes carbonyles ;
R₃ et R'₃ représentent chacun indépendamment un atome d'hydrogène ou d'halogène, un groupe -(C₁-C₆)Alk, hydroxy, -CN, -(C₁-C₆)alcoxy, -COR₄ ou -Y-CR₈R₉-COR₄;
Y représente O ou CH₂;
R₄ représente un groupe hydroxy, (C₁-C₆)alcoxy ou -NR₅R₆;
R₅ et R₆ représentent indépendamment un atome d'hydrogène; un groupe -(C₁-C₄)Alk; phényle, naphtyle ou pyridyle éventuellement substitués par un groupe R₇;
ou bien R₅ et R₆, avec l'atome d'azote qui les porte, forment un cycle de 5 à 7 atomes, saturé ou insaturé, éventuellement substitué par un groupe R₇; et
R₇ représente un atome d'hydrogène ou d'halogène, un groupe hydroxy, -(C₁-C₄)Alk, -(C₁-C₆)alcoxy, -NH(C₁-C₄)Alk, -N(C₁-C₄)Alk₂, -COO(C₁-C₄)Alk, benzyle, naphtylméthyle ou pyridylméthyle;
R₈ et R₉ représentent indépendamment un atome d'hydrogène ou un groupe -(C₁-C₄)Alk;
Alk désigne des radicaux monovalents d'un hydrocarbure à chaîne saturée droite ou ramifiée;
et leurs sels ou solvates.

2. Composé selon la revendication 1, où la configuration du carbone de la propanolamine portant le groupe OH est (S).

3. Composé selon la revendication lou 2, **caractérisé en ce que** A est un groupe de formule (a).

4. Composé selon l'une des revendications 1 à 3,**caractérisé en ce que** R est un groupe-NHSO₂(C₁-C₄)Alk.

5. Composé selon l'une des revendications 1 à 4 **caractérisé en ce que** R₁ est un atome d'hydrogène.

6. Composé selon l'une des revendications 1 à 5 **caractérisé en ce que** R₃ et/ou R'₃ sont un atome d'hydrogène, un groupe hydroxy, -(C₁-C₆)alcoxy ou -Y-CR₈R₉-COOCH₂-CH₃, R₈ étant tel que défini dans la revendication 1.

7. Composé choisi parmi:
N-[2-benzyloxy-5-({(2S)-2-hydroxy-3-[(1,2,3,4-tétra-hydro-2-naphtalènylméthyl)-amino]-propyl}-oxy)-phényl]-méthanesulfonamide;
N-[2-hydroxy-5-({(2S)-2-hydroxy-3-[(1,2,3,4-tétra-hydro-2-naphtalènylméthyl)-amino]-propyl}-oxy)-phényl]-méthanesulfonamide;
N- {2-benzyloxy-5-[((2S)-2-hydroxy-3- {[(7-méthoxy-1,2,3,4-tétra-hydro-2-naphtalènyl)-méthyl]-amino}-propyl)-oxy]-phényl}-méthanesulfonamide;
N-{2-hydroxy-5-[((2S)-2-hydroxy-3-{[(7-méthoxy-1,2,3,4-tétra-hydro-2-naphtalènyl)-méthyl]-amino}-propyl)-oxy]-phényl}-méthanesulfonamide;
N-{2-benzyloxy-5-[((25)-2-hydroxy-3-{[(6-méthoxy-1,2,3,4-tétra-hydro-2-naphtalènyl)-méthyl]-amino}-propyl)-oxy]-phényl}-méthanesulfonamide;
N-{(2-hydroxy-5-[((2S)-2-hydroxy-3-{[(6-méthoxy-1,2,3,4-tétra-hydro-2-naphtalènyl)-méthyl]-amino}-propyl)-oxy]-phényl}-méthanesulfonamide;
N-(2-benzyloxy-5-{[(2S)-2-hydroxy-3-({[(2R)-6-méthoxy-1,2,3,4-tétra-hydro-2-naphtalènyl]-méthyl} -amino)-propyl]-oxy}-phényl)-méthanesulfonamide;
N-(2-hydroxy-5-{[(2S)-2-hydroxy-3-({[(2R)-6-méthoxy-1,2,3,4-tétra-hydro-2-naphtalènyl]-méthyl}-amino)-propyl]-oxy}-phényl)-méthanesulfonamide;
N-{2-benzyloxy-5-[((2S)-2-hydroxy-3-{[(7-hydroxy-1,2,3,4-tétra-hydro-2-naphtalènyl)-méthyl]-amino }-propyl)-oxy]-phényl }-méthanesulfonamide;
N-{2-hydroxy-5-[((2S)-2-hydroxy-3-{[(7-hydroxy-1,2,3,4-tétra-hydro-2-naphtalènyl)-méthyl]-amino}-propyl)-oxy]-phényl}-méthanesulfonamide;
N-{2-benzyloxy-5-[((2S)-2-hydroxy-3-{[(6-hydroxy-1,2,3,4-tétra-hydro-2-naphtalènyl)-méthyl]-amino}-propyl)-oxy]-phényl}-méthanesulfonamide;
N-{2-hydroxy-5-[((2S)-2-hydroxy-3-{[(6-hydroxy-1,2,3,4-tétra-hydro-2-naphtalènyl)-méthyl]-amino}-propyl)-oxy]-phényl}-méthanesulfonamide;
(2S)-1-[4-benzyloxy-phénoxy]-3-{[(6-méthoxy-1,2,3,4-tétra-hydro-2-naphtalènyl)-méthyl]-amino 1-2-propanol;
4-[((2S)-2-hydroxy-3-{[(6-méthoxy-1,2,3,4-tétra-hydro-2-naphtalènyl)-méthyl]-amino}-propyl)-oxy]-phénol;
[(6-{[((2S)-3-{4-benzyloxy-3-[(méthylsulfonyl)-amino]phénoxy}-2-hydroxy-propyl)-amino]-méthyl}-5,6,7,8-tétra-hydro-2-naphtalènyl)-oxy]-acétate d'éthyle;
[(6- {[((2S)-2-hydroxy-3-{4-hydroxy-3-[(méthylsulfonyl)-amino]-phénoxy}-propyl)-amino]-méthyl}-5,6,7,8-tétra-hydro-2-naphtalènyl)-oxy]-acétate d'éthyle;
N-{2-benzyloxy-5-[((2S)-2-hydroxy-3-{[(5-méthoxy-1,2,3,4-tétra-hydro-2-naphtalènyl)-méthyl]-amino}-propyl)-oxy]-phényl}-méthanesulfonamide;
N-{2-hydroxy-5-[((2S)-2-hydroxy-3-{[(5-méthoxy-1,2,3,4-tétra-hydro-2-naphtalènyl)-méthyl]-amino}-propyl)-oxy]-phényl}-méthanesulfonamide;
N-{2-benzyloxy-5-[((2S)-2-hydroxy-3-{[(5-hydroxy-1,2,3,4-tétra-hydro-2-naphtalènyl)-méthyl]-amino}-propyl)-oxy]-phényl}-méthanesulfonamide;
N-{2-hydroxy-5-[((2S)-2-hydroxy-3-{[(5-hydroxy-1,2,3,4-tétra-hydro-2-naphtalènyl)-méthyl]-amino} -propyl)-oxy]-phényl }-méthanesulfonamide;
N-[2-benzyloxy-5-({(2S)-3-[(2,3,6,7,8,9-hexahydronaphto[2,3-b][1,4]dioxin-7-ylméthyl)-amino]-2-hydroxypropyl}-oxy)-phényl]-méthanesulfonamide;
N-[5-({(2S)-3-[(2,3,6,7,8,9-hexahydronaphto[2,3-b][1,4]dioxin-7-ylméthyl)-amino]-2-hydroxypropyl}-oxy)-2-hydroxyphényl]-méthanesulfonamide;
N-{2-benzyloxy-5-[((25)-2-hydroxy-3-[(7-méthoxy-1,2,3,4-tétra-hydro-1-naphtalènyl)-méthyl]-amino}-propyl)-oxy]-phényl}-méthanesulfonamide;
N-{2-hydroxy-5-[((2S)-2-hydroxy-3-{[(7-méthoxy-1,2,3,4-tétra-hydro-1-naphtalènyl)-méthyl]-amino}-propyl)-oxy]-phényl}-méthanesulfonamide;
N-{2-benzyloxy-5-[((2S)-2-hydroxy-3-[(7-hydroxy-1,2,3,4-tétra-hydro-1-naphtalènyl)-méthyl]-amino}-propyl)-oxy]-phényl}méthanesulfonamide;
N-{2-hydroxy-5-[((2S)-2-hydroxy-3-{[(7-hydroxy-1,2,3,4-tétra-hydro-1-naphtalènyl)-méthyl]-amino}-propyl)-oxy]-phényl}-méthanesulfonamide;
N-{2-benzyloxy-5-[((25)-3-{[(6,7-diméthoxy-1,2,3,4-tétra-hydro-1-naphtalènyl)-méthyl]-amino}-2-hydroxypropyl)oxy]phényl}-méthanesulfonamide;
N-{5-[((2S)-3-{[(6,7-diméthoxy-1,2,3,4-tétra-hydro-1-naphtalènyl)-méthyl]-amino}-2-hydroxypropyl)-oxy]-2-hydroxyphényl}-méthanesulfonamide;
leurs sels et leurs solvates.

8. Procédé de préparation des composés de formule (I) selon les revendications de 1 a 7 comprenant les étapes consistant à faire réagir un composé de formule (II) 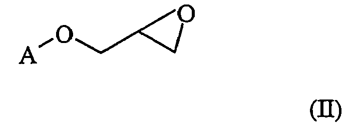 dans laquelle A est un groupe tel que défini dans la revendication 1,
avec une amine de formule (III) 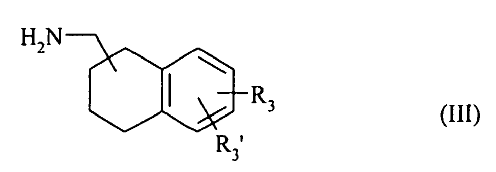 sou forme de base non salifiée, où R₃ et R'₃ sont tels que définis dans la revendication 1,
et éventuellement transformer le composé de formule (I) ainsi obtenu en un de ses sels.

9. Composé de formule (III') 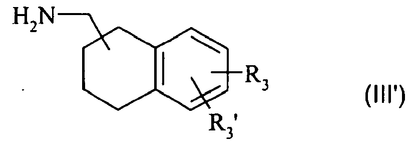 dans laquelle R₃ et R'₃ sont indépendamment un groupe -(CH₂)₂COR₄, R₄ étant tel que défini dans la revendication 1, ainsi que leurs sels.

10. Composition pharmaceutique contenant en tant que principe actif un composé de formule (I) selon l'une des revendications 1 à 7 ou l'un de ses sels pharmaceutiquement acceptables.

11. Médicament contenant en tant que principe actif un composé de formule (I) selon l'une des revendications 1 à 7 ou l'un de ses sels pharmaceutiquement acceptables.

12. Utilisation d'un composé de formule (I) ou un de ses sels ou solvates selon la revendication 1, pour la préparation d'un médicament destiné au traitement des maladies gastro-intestinales, de maladies du système nerveux central, de troubles du système urinaire, comme anti-obésité, anti-diabétique, psychotrope, anti-glaucomateux, cicatrisant, anti-dépresseur, comme inhibiteur des contractions utérines, comme tocolytiques pour prévenir ou retarder les accouchements précoces ou pour le traitement et/ou la prophylaxie de la dysménorrhée.

13. Réactif utilisable dans les essais biochimiques, qui comprend au moins un composé de formule (I) convenablement marqué.

## Claims

1. Compound of general formula (I): 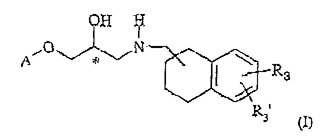 in which:
A is a group of formula (a) or (b) 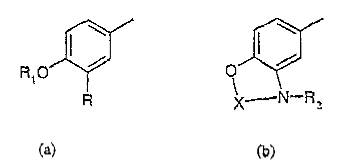
where
R represents a hydrogen or halogen atom, an -S(O)₂(C₁-C₄)alk group where z is 0, 1 or 2, an -NHSO₂(C₁-C₄) alk, -SO₂NH(C₁-C₄)alk, -NHSO₂-(C₁-C₄)alk-phenyl or -NHSO₂-phenyl group, it being possible for said phenyl to carry a halogen atom, a (C₁-C₄) alk group or a (C₁-C₆)alkoxy group;
R₁ represents a hydrogen atom or a - (C₁-C₉) alk, -CO(C₁-C₄) alk, -(C₁-C₄) alk-phenyl or -CO-phenyl group, it being possible for said phenyl to carry a halogen atom or a -(C₁-C₄)alk or (C₁-C₆)alkoxy group;
R₂ is a hydrogen atom, an -SO₂(C₁-C₄)alk, -SO₂-(C₁-C₄)alk-phenyl, -SO₂-phenyl, or a -(C₁-C₄)alk group;
X completes a saturated or unsaturated 5- to 8-atom ring which can carry one or two -(C₁-C₄) alk groups and/or one or two carbonyl groups;
R₃ and R'₃ each represent independently a hydrogen or halogen atom, a -(C₁-C₆)alk, hydroxyl, -CN, -(C₁-C₆) alkoxy, -COR₄ or -Y-CR₈R₉-COR₄ group;
Y represents O or CH₂;
R₄ represents a hydroxyl, (C₁-C₆)alkoxy or -NR₅R₆ group;
R₅ and R₆ represent independently a hydrogen atom; a -(C₁-C₄)alk, phenyl, naphthyl or pyridyl group optionally substituted with an R₇ group;
or else R₅ and R₆, with the nitrogen atom carrying them, form a saturated or unsaturated 5- to 7-atom ring optionally substituted with an R₇ group; and
R₇ represents a hydrogen or halogen atom, a hydroxyl, -(C₁-C₄)alk, -(C₁-C₆)alkoxy, -NH(C₁-C₄) alk, -N(C₁-C₄) alk₂, -COO(C₁-C₄)alk, benzyl, naphthylmethyl or pyridylmethyl group;
R₈ and R₉ represent independently a hydrogen atom or a (C₁-C₄) alk group;
alk designates monovalent radicals of a hydrocarbon having a saturated linear or branched chain;
and their salts or solvates.

2. Compound according to Claim 1, where the configuration of the carbon of the propanolamine carrying the OH group is (S).

3. Compound according to Claim 1 or 2, **characterized in that** A is a group of formula (a).

4. Compound according to one of Claims 1 to 3, **characterized in that** R is an -NHSO₂(C₁-C₄)alk group.

5. Compound according to one of Claims 1 to 4, **characterized in that** R₁ is a hydrogen atom.

6. Compound according to one of Claims 1 to 5, **characterized in that** R₃ and/or R'₃ are a hydrogen atom, a hydroxyl, -(C₁-C₆)alkoxy or -Y-CR₈R₉-COOCH₂-CH₃ group, R₈ being as defined in claim 1.

7. Compound selected from:
N-[2-Benzyloxy-5-({(2S)-2-hydroxy-3-[(1,2,3,4-tetrahydro-2-naphthalenylmethyl)amino]propyl}oxy)-phenyl]methanesulfonamide;
N-[2-Hydroxy-5-({(2S)-2-hydroxy-3-[(1,2,3,4-tetrahydro-2-naphthalenylmethyl)amino]propyl}oxy)-phenyl]methanesulfonamide;
N-{2-Benzyloxy-5-[((2S)-2-hydroxy-3-{[(7-methoxy-1,2,3,4-tetrahydro-2-naphthalenyl)methyl]amino}-propyl)oxy]phenyl}methanesulfonamide;
N-{2-Hydroxy-5-[((2S)-2-hydroxy-3-{[(7-methoxy-1,2,3,4-tetrahydro-2-naphthalenyl)methyl]amino}-propyl)oxy]phenyl}methanesulfonamide;
N-{2-Benzyloxy-5-[((2S)-2-hydroxy-3-{[(6-methoxy-1,2,3,4-tetrahydro-2-naphthalenyl)methyl]amino}-propyl)oxy]phenyl}methanesulfonamide;
N-{2-Hydroxy-5-[((2S)-2-hydroxy-3-{[(6-methoxy-1,2,3,4-tetrahydro-2-naphthalenyl)methyl]amino}-propyl)oxy]phenyl}methanesulfonamide;
N-(2-Benzyloxy-5-{[(2S)-2-hydroxy-3-({[(2R)-6-methoxy-1,2,3,4-tetrahydro-2-naphthalenyl]methyl}amino)propyl]oxy}phenyl)-methanesulfonamide:
N-(2-Hydroxy-5-{[(2S)-2-hydroxy-3-({[(2R)-6-methoxy-1,2,3,4-tetrahydro-2-naphthalenyl]methyl}amino)propyl]oxy}-phenyl}methanesulfonamide;
N-{2-Benzyloxy-5-[((2S)-2-hydroxy-3-{[(7-hydroxy-1,2,3,4-tetrahydro-2-naphthalenyl)methyl]amino}-propyl)oxylphenyl}methanesulfonamide;
N-{2-Hydroxy-5-[((2S)-2-hydroxy-3-{[(7-hydroxy-1,2,3,4-tetrahydro-2-naphthalenyl)methyl]amino}-propyl)oxy]phenyl}methanesulfonamide;
N-{2-Benzyloxy-5-[((2S)-2-hydroxy-3-{[(6-hydroxy-1,2,3,4-tetrahydro-2-naphthalenyl)methyl]amino}-propyl)oxy]phenyl}methanesulfonamide;
N-{2-Hydroxy-5-[((2S)-2-hydroxy-3-{[(6-hydroxy-1,2,3,4-tetrahydro-2-naphthalenyl)methyl]amino}-propyl)oxy]phenyl}methanesulfonamide;
(2S)-1-[4-Benzyloxyphenoxy]-3-{[(6-methoxy-1,2,3,4-tetrahydro-2-naphthalenyl)methyl]amino}-2-propanol;
4-[((2S)-2-Hydroxy-3-{[(6-methoxy-1,2,3,4-tetrahydro-2-naphthalenyl)methyl]amino}propyl)oxy]phenol;
Ethyl [(6-{[((2S)-3-{4-benzyloxy-3-[(methylsulfonyl)amino]phenoxy}-2-hydroxypropyl)amino]-methyl}-5,6,7,8-tetrahydro-2-naphthalenyl)oxy]acetate;
Ethyl [(6-{[((2S)-2-hydroxy-3-{4-hydroxy-3-[(methylsulfonyl)amino]-phenoxy}propyl)amino]methyl}-5,6,7,8-tetrahydro-2-naphthalenyl)oxy]acetate;
N-{2-Benzyloxy-5-[((2S)-2-hydroxy-3-{[(5-methoxy-1,2,3,4-tetrahydro-2-naphthalenyl)methyl]amino}-propyl)oxy]phenyl}methanesulfonamide:
N-{2-Hydroxy-5-[((2S)-2-hydroxy-3-{[(5-methoxy-1,2,3,4-tetrahydro-2-naphthalenyl)methyl]amino}-propyl)oxy]phenyl}methanesulfonamide;
N-{2-Benzyloxy-5-[((2S)-2-hydroxy-3-{[(5-hydroxy-1,2,3,4-tetrahydro-2-naphthalenyl)methyl]amino}-propyl)oxy]phenyl}methanesulfonamide;
N-{2-Hydroxy-5-[((2S)-2-hydroxy-3-{[(5-hydroxy-1,2,3,4-tetrahydro-2-naphthalenyl)methyl]amino}-propyl)oxy]phenyl}methanesulfonamide;
N-[2-Benzyloxy-5-({(2S)-3-[(2,3,6,7,8,9-hexahydronaphtho[2,3-b][1,4]dioxin-7-ylmethyl)amino]-2-hydroxypropyl}oxy)phenyl]methanesulfonamide;
N-[5-({(2S)-3-[(2,3,6,7,8,9-Hexahydronaphtho-[2,3-b] [1,4]dioxin-7-ylmethyl)amino]-2-hydroxypropyl}oxy)-2-hydroxyphenyl]methanesulfonamide;
N-{2-Benzyloxy-5-[((2S)-2-hydroxy-3-{[(7-methoxy-1,2,3,4-tetrahydro-1-naphthalenyl)methyl]amino}-propyl)oxy]phenyl}methanesulfonamide;
N-{2-Hydroxy-5-[((2S)-2-hydroxy-3-{[(7-methoxy-1,2,3,4-tetrahydro-1-naphthalenyl)methyl]amino}-propyl)oxylphenyl}methanesulfonamide;
N-{2-Benzyloxy-5-[((2S)-2-hydroxy-3-{[(7-hydroxy-1,2,3,4-tetrahydro-1-naphthalenyl)methyl]amino}-propyl)oxy]phenyl}methanesulfonamide;
N-{2-Hydroxy-5-[((2S)-2-hydroxy-3-{[(7-hydroxy-1,2,3,4-tetrahydro-1-naphthalenyl)methyl]amino}-propyl)oxy]phenyl}methanesulfonamide;
N-{2-Benzyloxy-5-[((2S)-3-{[(6,7-dimethoxy-1,2,3,4-tetrahydro-1-naphthalenyl)methyl]amino}-2-hydroxypropyl)oxy]phenyl}methanesulfonamide;
N-{5-[((2S)-3-{[(6,7-Dimethoxy-1,2,3,4-tetrahydro-1-naphthalenyl) methyl]amino}-2-hydroxypropyl)oxy]-2-hydroxyphenyl}methanesulfonamide; their salts and their solvates.

8. Process for preparing the compounds of formula (I) according to Claims 1 to 7 comprising the steps consisting of
reacting a compound of formula (II) 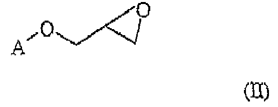 in which A is a group as defined in Claim 1,
with an amine of formula (III) 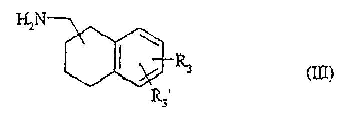 in the form of a nonsalified base, where R₃ and R'₃ are as defined in Claim 1,
and optionally converting the compound of formula (I) thus obtained into one of its salts.

9. Compound of formula (III') 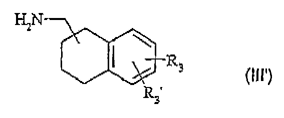 in which R₃ and R'₃ are independently a - (CH₂)₂COR₄ group, R₄ being as defined in Claim 1, and their salts.

10. Pharmaceutical composition containing a compound of formula (I) according to one of Claims 1 to 7 or one of its pharmaceutically acceptable salts as the active principle.

11. Drug containing a compound of formula (I) according to one of Claims 1 to 7 or one of its pharmaceutically acceptable salts as the active principle.

12. Use of a compound of formula (I) or one of its salts or solvates according to Claim 1 in the preparation of a drug intended for the treatment of gastrointestinal diseases, disorders of the central nervous system, problems with the urinary system, as anti-obesity agent, as anti-diabetic agent, as psychotropic agent, as anti-glaucoma agent, as cicatrizing agent, as anti-depressant, as a urine contraction inhibitor, as tocolytics to prevent or retard premature birth, or in the treatment and/or prophylaxis of dysmenorrhoea.

13. Reagent that can be used in biochemical tests, which comprises at least one suitably labelled compound of formula (I).

## Patentansprüche

1. Verbindung der allgemeinen Formel (I) 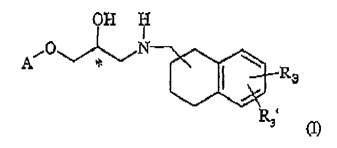 worin
A für eine Gruppe der Formel (a) oder (b) steht 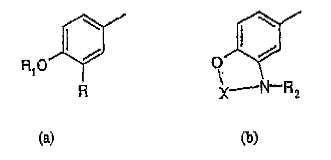
worin
R für ein Wasserstoff- oder Halogenatom, eine -S(O)_{z}(C₁-C₄)-Alk-Gruppe, worin z für 0, 1 oder 2 steht, oder eine -NHSO₂(C₁-C₄)-Alk-, -SO₂NH(C₁-C₄)-Alk-, -NHSO₂-(C₁-C₄)-Alk-Phenyl- oder -NHSO₂-Phenylgruppe steht, wobei das Phenyl ein Halogenatom, eine (C₁-C₄)-Alk- oder eine (C₁-C₆)-Alkoxygruppe tragen kann;
R₁ für ein Wasserstoffatom oder eine -(C₁-C₄)-Alk-, -CO(C₁-C₄)-Alk-, -(C₁-C₄)-Alk-Phenyl- oder -CO-Phenylgruppe steht, wobei das Phenyl ein Halogenatom, eine (C₁-C₄)-Alk- oder eine (C₁-C₆)-Alkoxygruppe tragen kann;
R₂ für ein Wasserstoffatom, eine -SO₂(C₁-C₄)-Alk-, -SO₂-(C₁-C₄)-Alk-Phenyl-, -SO₂-Phenylgruppe oder eine -(C₁-C₄)-Alk-Gruppe steht;
X einen gesättigten oder ungesättigten Ring mit 5 bis 8 Atomen, der eine oder zwei -(C₁-C₄)-Alk-Gruppen und/oder eine oder zwei Carbonylgruppen tragen kann, vervollständigt;
R₃ und R'₃ jeweils unabhängig voneinander für ein Wasserstoff- oder Halogenatom oder eine -(C₁-C₆)-Alk-, Hydroxy-, -CN-, -(C₁-C₆)-Alkoxy-, -COR₄- oder -Y-CR₈R₉-COR₄-Gruppe stehen;
Y für O oder CH₂ steht;
R₄ für eine Hydroxy-, (C₁-C₆)-Alkoxy- oder -NR₅R₆-Gruppe steht;
R₅ und R₆ unabhängig voneinander für ein Wasserstoffatom, eine -(C₁-C₄)-Alk-Gruppe oder eine Phenyl-, Naphthyl- oder Pyridylgruppe, die gegebenenfalls durch eine Gruppe R_{7'} substituiert ist, stehen;
oder R₅ und R₆ mit dem Stickstoffatom, an das sie gebunden sind, einen gesättigten oder ungesättigten Ring mit 5 bis 7 Atomen, der gegebenenfalls durch eine Gruppe R_{7'} substituiert ist, bilden; und
R₇ für ein Wasserstoff- oder Halogenatom oder eine Hydroxy-, -(C₁-C₄)-Alk-, -(C₁-C₆)-Alkoxy-, -NH(C₁-C₄)-Alk-, -N(C₁-C₄) -Alk₂-, -COO(C₁-C₄)-Alk-, Benzyl-, Naphthylmethyl- oder Pyridylmethylgruppe steht;
R₈ und R₉ unabhängig voneinander für ein Wasserstoffatom oder eine (C₁-C₄) -Alk-Gruppe stehen;
Alk für einwertige Reste eines gerad- oder verzweigtkettigen Kohlenwasserstoffs steht;
und Salze oder Solvate davon.

2. Verbindung nach Anspruch 1, wobei der die OH-Gruppe tragende Kohlenstoff des Propanolamins (S)-Konfiguration aufweist.

3. Verbindung nach Anspruch 1 oder 2, **dadurch gekennzeichnet, daß** A für eine Gruppe der Formel (a) steht.

4. Verbindung nach einem der Ansprüche 1 bis 3, **dadurch gekennzeichnet, daß** R für eine -NHSO₂(C₁-C₄)-Alk-Gruppe steht.

5. Verbindung nach einem der Ansprüche 1 bis 4, **dadurch gekennzeichnet, daß** R₁ für ein Wasserstoffatom steht.

6. Verbindung nach einem der Ansprüche 1 bis 5, **dadurch gekennzeichnet, daß** R₃ und/oder R'₃ für ein Wasserstoffatom oder eine Hydroxy-, -(C₁-C₆)-Alkoxy- oder -Y-CR₆R₉-COOCH₂-CH₃-Gruppe, wobei R₈ die in Anspruch 1 angegebene Bedeutung besitzt, stehen.

7. Verbindung, ausgewählt unter:
N-[2-Benzyloxy-5-({(25)-2-hydroxy-3-[(1,2,3,4-tetrahydro-2-naphthalinylmethyl)amino]propyl}oxy)-phenyl]methansulfonamid;
N-[2-Hydroxy-5-({(2S)-2-hydroxy-3-[(1,2,3,4-tetrahydro-2-naphthalinylmethyl)amino]propyl}oxy)-phenyl]methansulfonamid;
N-{2-Benzyloxy-5-[((2S)-2-hydroxy-3-{[(7-methoxy-1,2,3,4-tetrahydro-2-naphthalinyl)methyl]amino}-propyl)oxy]phenyl}methansulfonamid;
N-{2-Hydroxy-5-[((2S)-2-hydroxy-3-{[(7-methoxy-1,2,3,4-tetrahydro-2-naphthalinyl)methyl]amino}-propyl)oxy]phenyl}methansulfonamid;
N-{2-Benzyloxy-5-[((2S)-2-hydroxy-3-{[(6-methoxy-1,2,3,4-tetrahydro-2-naphthalinyl)methyl]amino}-propyl)oxy]phenyl}methansulfonamid;
N-{2-Hydroxy-5-[((2S)-2-hydroxy-3-{[(6-methoxy-1,2,3,4-tetrahydro-2-naphthalinyl)methyl]amino}-propyl)oxy]phenyl}methansulfonamid;
N-(2-Benzyloxy-5-{[(2S)-2-hydroxy-3-({[(2R)-6-methoxy-1,2,3,4-tetrahydro-2-naphthalinyl]methyl}-amino)propyl]oxy}phenyl)methansulfonamid;
N-(2-Hydroxy-5-{[(2S)-2-hydroxy-3-({[(2R)-6-methoxy-1,2,3,4-tetrahydro-2-naphthalinyl]methyl}-amino)propyl]oxy}phenyl}methansulfonamid;
N-{2-Benzyloxy-5-[((2S)-2-hydroxy-3-{[(7-hydroxy-1,2,3,4-tetrahydro-2-naphthalinyl)methyl]amino}-propyl)oxylphenyl}methansulfonamid;
N-{2-Hydroxy-5-[((2S)-2-hydroxy-3-{[(7-hydroxy-1,2,3,4-tetrahydro-2-naphthalinyl)methyl]amino}-propyl)oxy]phenyl}methansulfonamid;
N-{2-Benzyloxy-5-[((2S)-2-hydroxy-3-{[(6-hydroxy-1,2,3,4-tetrahydro-2-naphthalinyl)methyl]amino}-propyl)oxy]phenyl}methansulfonamid;
N-{2-Hydroxy-5-[((2S)-2-hydroxy-3-{[(6-hydroxy-1,2,3,4-tetrahydro-2-naphthalinyl)methyl]amino}-propyl)oxy]phenyl}methansulfonamid;
(2S)-1-[4-Benzyloxyphenoxy]-3-{[(6-methoxy-1,2,3,4-tetrahydro-2-naphthalinyl)methyl]amino}-2-propanol;
4-[((2S)-2-Hydroxy-3-{[(6-methoxy-1,2,3,4-tetrahydro-2-naphthalinyl)methyl]amino}propyl)oxy]-phenol;
[(6-{[((2S)-3-{4-Benzyloxy-3-[(methylsulfonyl)-amino]phenoxy}-2-hydroxypropyl)amzno]methyl}-5,6,7,8-tetrahydro-2-naphthalinyl)oxy]essigsäureethylester;
[(6-{[((2S)-2-Hydroxy-3-{4-hydroxy-3-[(methylsulfonyl)amino]phenoxy}propyl)amino]methyl}-5,6,7,8-tetrahydro-2-naphthalinyl)oxy]essigsäureethylester;
N-{2-Benzyloxy-5-[((2S)-2-hydroxy-3-{[(5-methoxy-1,2,3,4-tetrahydro-2-naphthalinyl)methyl]amino}-propyl)oxy]phenyl}methansulfonamid;
N-{2-Hydroxy-5-[((2S)-2-hydroxy-3-{[(5-methoxy-1,2,3,4-tetrahydro-2-naphthalinyl)methyl]amino}-propyl)oxy]phenyl}methansulfonamid;
N-{2-Benzyloxy-5-[((2S)-2-hydroxy-3-{[(5-hydroxy-1,2,3,4-tetrahydro-2-naphthalinyl)methyl]amino}-propyl)oxy]phenyl}methansulfonamid;
N-{2-Hydroxy-5-[((2S)-2-hydroxy-3-{[(5-hydroxy-1,2,3,4-tetrahydro-2-naphthalinyl)methyl]amino}-propyl)oxy]phenyl}methansulfonamid;
N-[2-Benzyloxy-5-({(2S)-3-[(2,3,6,7,8,9-hexahydronaphtho[2,3-b][1,4]dioxin-7-ylmethyl)amino]-2-hydroxypropyl}oxy)phenyl]methansulfonamid;
N-[5-({(2S)-3-[(2,3,6,7,8,9-Hexahydronaphtho-[2,3-b][1,4]dioxin-7-ylmethyl)amino]-2-hydroxypropyl}oxy)-2-hydroxyphenyl]methansulfonamid;
N-{2-Benzyloxy-5-[((2S)-2-hydroxy-3-{[(7-methoxy-1,2,3,4-tetrahydro-1-naphthalinyl)methyl]amino}-propyl)oxy]phenyl}methansulfonamid;
N-{2-Hydroxy-5-[((2S)-2-hydroxy-3-{[(7-methoxy-1,2,3,4-tetrahydro-1-naphthalinyl)methyl]amino}-propyl)oxy]phenyl}methansulfonamid:
N-{2-Benzyloxy-5-[((2S)-2-hydroxy-3-{[(7-hydroxy-1,2,3,4-tetrahydro-1-naphthalinyl)methyl]amino}-propyl)oxy]phenyl}methansulfonamid;
N-{2-Hydroxy-5-[((2S)-2-hydroxy-3-{[(7-hydroxy-1,2,3,4-tetrahydro-1-naphthalinyl)methyl]amino}-propyl)oxylphenyl}methansulfonamid;
N-{2-Benzyloxy-5-[((2S)-3-{[(6,7-dimethoxy-1,2,3,4-tetrahydro-1-naphthalinyl)methyl]amino}-2-hydroxypropyl)oxy]phenyl}methansulfonamid;
N-{5-[((2S)-3-{[(6,7-Dimethoxy-1,2,3,4-tetrahydro-1-naphthalinyl)methyl]amino}-2-hydroxypropyl)oxy]-2-hydroxyphenyl}methansulfonamid;
Salzen und Solvaten davon.

8. Verfahren zur Herstellung von Verbindungen der Formel (I) nach den Ansprüchen 1 bis 7, bei dem man
eine Verbindung der Formel (II) 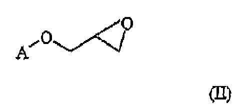 worin A für eine wie in Anspruch 1 definierte Gruppe steht,
mit einem Amin der Formel (III) 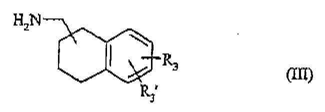 in unversalzter Basenform, wobei R₃ und R'₃ die in Anspruch 1 angegebene Bedeutung besitzen, umsetzt und gegebenenfalls die so erhaltene Verbindung der Formel (I) in eines ihrer Salze umwandelt.

9. Verbindung der Formel (III') 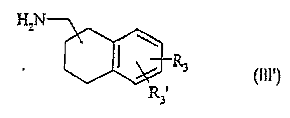 worin R₃ und R'₃ unabhängig voneinander für eine -(CH₂)₂COR₄-Gruppe stehen, wobei R₄ die in Anspruch 1 angegebene Bedeutung besitzt, sowie Salze davon.

10. Pharmazeutische Zusammensetzung, enthaltend als Wirkstoff eine Verbindung der Formel (I) nach einem der Ansprüche 1 bis 7 oder ein pharmazeutisch unbedenkliches Salz davon.

11. Arzneimittel, enthaltend als Wirkstoff eine Verbindung der Formel (I) nach einem der Ansprüche 1 bis 7 oder ein pharmazeutisch unbedenkliches Salz davon.

12. Verwendung einer Verbindung der Formel (I) oder eines Salzes oder Solvats davon nach Anspruch 1 zur Herstellung eines Arzneimittels zur Behandlung von Magen-Darm-Erkrankungen, Erkrankungen des Zentralnervensystems, Beschwerden des Harnapparats, Antiadiposita, Antidiabetika, Psychotropika, Antiglaukomata, Vernarbungsmittel, Antidepressiva, als Uteruskontraktionshemmer, als Tocolytika zur Prävention oder Verlangsamung von vorzeitigen Wehen oder zur Behandlung und/oder Prophylaxe von Dysmenorrhoe.

13. Bei biochemischen Assays verwendbares Reagens, das mindestens eine zweckmäßig markierte Verbindung der Formel (I) umfaßt.
